# EUROPEAN PATENT APPLICATION

(11) **EP 1 862 445 A1**
(43) Date of publication of application: **05.12.2007**
(21) Application number: 06729087.4
(22) Date of filing: 14.03.2006
(51) Int. Cl.: C07C 51/47, C07C 51/265, C07C 63/26, C07C 63/04, C07C 63/06

(54) **PROCESS FOR PRODUCING HIGH-PURITY TEREPHTHALIC ACID**

(30) Priority: 22.03.2005 JP 2005082038; 23.03.2005 JP 2005084186
(71) Applicant: Mitsubishi Chemical Corporation, Minato-ku Tokyo 108-0014 (JP)
(72) Inventor: NUMATA, Motoki, MITSUBISHI CHEMICAL CORPORATION, Kitakyushu-shi, Fukuoka 8060004 (JP); TAKAHASHI, Isao, MITSUBISHI CHEMICAL CORPORATION, Kitakyushu-shi, Fukuoka, 8060004 (JP)
(74) Representative: Vossius & Partner
(86) International application number: PCT/JP2006/305053
(87) International publication number: WO 2006/100969

(57) **Abstract**

A problem of the invention is to provide a process of producing high purity terephthalic acid by selectively recovering p-toluic acid in wastewater which has hitherto been discarded and using it as a raw material of terephthalic acid.

The invention is concerned with a process of producing high purity terephthalic acid, which is **characterized in that** a p-toluic acid recovery step of recovering p-toluic acid from a primary mother liquor and feeding it into an oxidation reaction step includes the following respective steps of: (I) an adsorption step of feeding, as a liquid to be treated, the primary mother liquor or a secondary mother liquor obtained by subjecting the primary mother liquor as cooled to solid-liquid separation into an adsorption tower filled with an adsorbing agent in which the breakthrough time of p-toluic acid is longer than the breakthrough time of benzoic acid to adsorb p-toluic acid and benzoic acid in the liquid to be treated to the adsorbing agent, (II) a feed stopping step of stopping the of the liquid to be treated into the adsorption tower at an arbitrary point of time of exceeding the breakthrough time of benzoic acid, (III) a desorption step of feeding a desorbing agent into the adsorption tower to desorb adsorbed p-toluic acid, and (IV) a circulation of feeding p-toluic acid contained in the desorbing agent flown out from the adsorption tower into the oxidation reaction step.

## Description

### TECHNICAL FIELD

The present invention relates to a process of producing high purity terephthalic acid. In detail, the invention relates to a process of producing high purity terephthalic acid by oxidation of p-xylene, wherein p-toluic acid formed by a by-product is recovered and fed into an oxidation step.

### BACKGROUND ART

At present, high purity terephthalic acid is produced on a large scale by a production process via respective steps including an oxidation reaction step of oxidizing p-xylene in an acetic acid solvent to form terephthalic acid; a crude terephthalic acid crystal obtaining step of obtaining crude terephthalic acid from a slurry obtained in the oxidation reaction step; a hydrogenation purification step of dissolving obtained crude terephthalic acid in hot water and hydrogenating it to achieve purification; a high purity terephthalic acid crystallization step of crystallizing high purity terephthalic acid from a solution after passing through the hydrogenation purification step; a high purity terephthalic acid crystal obtaining step of recovering crystallized high purity terephthalic acid; and a washing and drying step of washing obtained high purity terephthalic acid with water and drying it.

Considerable amounts of organic substances as water-soluble by-products, for example, p-toluic acid and benzoic acid are contained in wastewater of these steps, especially the high purity terephthalic acid crystal obtaining step, namely a mother liquor after the crystal separation. If this wastewater is discharged into the environment as it stands, pollution of the environment is caused, and, therefore, it must be discharged after reducing a concentration of the organic substances by a wastewater treatment apparatus. This treatment requires a huge cost.

Then, Patent Document 1 proposes a method in which after removing insoluble organic substances in the wastewater by filtration by a filter, heavy metals are removed by an ion exchange resin and dissolved organic substance salts recovered by a reverse osmosis system.

Also, Non-Patent Document 1 proposes that this wastewater, namely the mother liquor after the crystal separation is treated with an adsorbing agent to remove organic substances such as p-toluic acid and benzoic acid and then reused as hot water for dissolving crude terephthalic acid in the hydrogenation purification step.
Patent Document 1: JP-T-2003-507156
Non-Patent Document 1: P.K. Khachane et al., Separation Science and Technology, Vol. 38, No. 1, pp. 93-111, 2003

### DISCLOSURE OF THE INVENTION

### PROBLEMS THAT THE INVENTION IS TO SOLVE

Now, though as described previously, considerable amounts of p-toluic acid and benzoic acid are contained in the wastewater, since p-toluic acid is converted to terephthalic acid through oxidation, if p-toluic acid in the wastewater can be recovered and fed into the oxidation reaction step, it becomes possible to enhance a yield of terephthalic acid.

However, in the method of Patent Document 1, p-toluic acid is recovered as a mixture with benzoic acid. Accordingly, if recovered p-toluic acid is fed into the oxidation reaction step, a concentration of benzoic acid in the reaction system is increased due to entrained benzoic acid, and a non-negligible amount of benzoic acid is contained in a high purity terephthalic acid product. If benzoic acid is contained in terephthalic acid, in producing a polyester by using this as a raw material, there is a possibility that a degree of polymerization of the resulting polyester does not reach a prescribed value; and there is a possibility that coloration of the polyester is caused. Accordingly, p-toluic acid recovered as a mixture with benzoic acid cannot be reused as a raw material of terephthalic acid as it stands. In consequence, a method of recovering only p-toluic acid efficiently from wastewater containing benzoic acid and p-toluic acid is being demanded.

### MEANS FOR SOLVING THE PROBLEMS

Non-Patent Document 1 describes that benzoic acid and p-toluic acid are adsorbed on a porous adsorbing agent from an aqueous solution and that adsorbed benzoic acid and p-toluic acid can be desorbed with acetic acid or methyl acetate. However, this document does not disclose that benzoic acid and p-toluic acid can be separated by using a porous adsorbing agent.

In feeding an aqueous solution containing both benzoic acid and p-toluic acid into an adsorption tower filled with this porous adsorbing agent, the present inventors studied the effluence state of these compounds into an effluent flown out from the adsorption tower. As a result, it has been found that at the beginning, all of benzoic acid and p-toluic acid are adsorbed and therefore, a liquid not containing any of the both flows out from the adsorption tower; however, when the feed of the aqueous solution is continued, benzoic acid becomes contained in the effluent and a concentration of benzoic acid in the effluent increases gradually, and when this concentration becomes considerably high, p-toluic acid becomes first contained in the effluent and its concentration increases gradually, leading to accomplishment of the invention.

This phenomenon reveals that p-toluic acid is stronger in adsorptivity to the porous adsorbing agent than benzoic acid and that after the adsorption tower has reached substantially saturated adsorption by benzoic acid and p-toluic acid, p-toluic acid displaces adsorbed benzoic acid and is adsorbed. Accordingly, by utilizing this phenomenon, if the feed of the aqueous solution is stopped to achieve desorption at a point of time at which adsorbed benzoic acid is thoroughly displaced with p-toluic acid and a ratio of p-toluic acid to benzoic acid within the adsorption tower is thoroughly larger than the ratio in the liquid, p-toluic acid can be selectively recovered.

Also, it has been found that since in the effluent flown out from the adsorption tower, p-toluic acid is reduced, if this can be reused as water for dissolving crude terephthalic acid or water for washing high purity terephthalic acid, process water can be reduced and a huge cost required when it is intended to discard this as it stands can be reduced, resulting in a reduction in the production cost of high purity terephthalic acid.

That is, a gist of the invention resides in the following (1) to (17).
(1) A process of producing high purity terephthalic acid including respective steps of (a) an oxidation reaction step of oxidizing p-xylene in an acetic acid solution where a catalyst is present to form terephthalic acid, (b) a crude terephthalic acid crystal obtaining step of subjecting a slurry in which formed terephthalic acid is deposited to solid-liquid separation to obtain a crude terephthalic acid crystal, (c) a hydrogenation step of dissolving the crude terephthalic acid crystal in water to form an aqueous solution and hydrogenating this, (d) a high purity terephthalic acid crystallization step of crystallizing terephthalic acid from the hydrogenated aqueous solution to form a high purity terephthalic acid slurry, (e) a high purity terephthalic acid crystal obtaining step of subjecting the high purity terephthalic acid slurry to solid-liquid separation to obtain a high purity terephthalic acid crystal and a primary mother liquor, and (f) a p-toluic acid recovery step of recovering p-toluic acid from the primary mother liquor and feeding it into the oxidation reaction step, which is characterized in that the p-toluic acid recovery step includes the following respective steps (I) to (IV):
   (I) an adsorption step of feeding, as a liquid to be treated, the primary mother liquor or a secondary mother liquor obtained by subjecting the primary mother liquor as cooled to solid-liquid separation into an adsorption tower filled with an adsorbing agent in which the breakthrough time of p-toluic acid is longer than the breakthrough time of benzoic acid to adsorb p-toluic acid and benzoic acid in the liquid to be treated to the adsorbing agent,
   (II) a feed stopping step of stopping the feed of the liquid to be treated into the adsorption tower at an arbitrary point of time of exceeding the breakthrough time of benzoic acid,
   (III) a desorption step of feeding a desorbing agent into the adsorption tower to desorb adsorbed p-toluic acid, and
   (IV) a circulation step of feeding p-toluic acid contained in the desorbing agent flown out from the adsorption tower into the oxidation reaction step.

(2) The process of producing high purity terephthalic acid as forth above in (1), which is characterized by further including the following respective steps (g) and (h):
   (g) a high purity terephthalic acid crystal washing and drying step of washing the high purity terephthalic acid crystal with water and then drying it to obtain a high purity terephthalic acid product, and
   (h) a washing wastewater circulation step of reusing washing wastewater in the high purity terephthalic acid crystal washing and drying step as water for dissolving the crude terephthalic acid crystal in the hydrogenation step (c).
(3) The process of producing high purity terephthalic acid as set forth above in (1) or (2) which is characterized by further including the following step (i):
   (i) an effluent circulation step of reusing at least a part of an effluent from the adsorption tower in the adsorption step (I) as water for dissolving the crude terephthalic acid crystal in the hydrogenation step (c).
(4) The process of producing high purity terephthalic acid as set forth above in (2), which is characterized by further including the following step (ii):
   (ii) an effluent circulation step of reusing at least a part of an effluent from the adsorption tower in the adsorption step (I) as water for washing the high purity terephthalic acid crystal in the high purity terephthalic acid crystal washing step (g).
(5) The process of producing high purity terephthalic acid as forth above in any one of (1) to (4), which is characterized by adjusting an amount of the effluent to be reused in the effluent circulation step (i) or (ii) such that the concentration of benzoic acid in the primary mother liquor in the high purity terephthalic acid crystal obtaining step (e) is not more than 3,000 ppm.

(6) The process of producing high purity terephthalic acid as set forth above in any one of (1) to (5), which is characterized by carrying out the crystallization of terephthalic acid in the high purity terephthalic acid crystallization step (d) by evaporating water from the aqueous solution to lower a temperature of the aqueous solution, condensing a generated water vapor and feeding this condensate as the liquid to be treated into the adsorption tower in the adsorption step (I).

(7) The process of producing high purity terephthalic acid as forth above in any one of (1) to (6), which is characterized by carrying out the feed stopping of the liquid to be treated into the adsorption tower in the feed stopping step (II) at a point of time at which the concentration of benzoic acid in the effluent from adsorption tower has reached at least 50% of the concentration of benzoic acid in the liquid to be treated.

(8) The process of producing high purity terephthalic acid as forth above in any one of (1) to (6), which is characterized by carrying out the feed stopping of the liquid to be treated into the adsorption tower in the feed stopping step (II) at or after the point of time at which the concentration of benzoic acid in the effluent from adsorption tower has equal to the concentration of benzoic acid in the liquid to be treated.

(9) The process of producing high purity terephthalic acid as forth above in any one of (1) to (8), which is characterized by carrying out the feed stopping of the liquid to be treated into the adsorption tower in the feed stopping step (II) before the concentration of p-toluic acid in the effluent from adsorption tower reaches 50% of the concentration of p-toluic acid in the liquid to be treated.

(10) The process of producing high purity terephthalic acid as set forth above in any of (1) to (8), which is characterized by carrying out the feed stopping of the liquid to be treated into the adsorption tower in the feed stopping step (II) before the concentration of p-toluic acid in the effluent from adsorption tower reaches 20% of the concentration of p-toluic acid in the liquid to be treated.

(11) The process of producing high purity terephthalic acid as forth above in any one of (1) to (10), which is characterized in that in the adsorption step (I), plural adsorption towers filled with an adsorbing agent in which the breakthrough time of p-toluic acid is longer than the breakthrough time of benzoic acid are provided, and the respective adsorption towers configured so as to repeat adsorption - desorption - regeneration, the primary mother liquor or the secondary mother liquor obtained by subjecting the primary mother liquor as cooled to solid-liquid separation is fed into a first adsorption tower and p-toluic acid and benzoic acid in the liquid to be treated are adsorbed to the adsorbing agent (however, the effluent of the first adsorption tower is discharged out an adsorption process system during a time when p-toluic acid does not flow out in the effluent) ; that in the feed stopping step (II), when p-toluic acid in the effluent from the first adsorption tower has reached a prescribed concentration, the feed of the liquid to be treated into the first adsorption tower is stopped, and a feed destination of the liquid to be treated is switched from the first adsorption tower to a second adsorption tower; that in the desorption step (III), the desorbing agent is into the first adsorption tower to desorb adsorbed p-toluic acid; and in the circulation step (IV), p-toluic acid contained in the desorbing agent flown out from the first adsorption tower is fed into the oxidation reaction step.

(12) The process of producing high purity terephthalic acid as forth above in (11), which is characterized by further feeding water into the first adsorption tower after passing through the desorption step (III) to elute the adsorbed desorbing agent and using the first adsorption tower as the adsorption tower in the adsorption step (I).

(13) The process of producing high purity terephthalic acid as set forth above in any one of (1) to (12), which is characterized in that the desorbing agent is acetic acid, methyl acetate or a mixture thereof.
(14) The process of producing high purity terephthalic acid as forth above in any one of (1) to (13), which is characterized in that the adsorbing agent is a porous copolymer of a monovinyl compound and a polyvinyl compound.

(15) The process of producing high purity terephthalic acid as set forth above in any one of (1) to (13), which is characterized in that the adsorbing agent is a porous copolymer containing styrene and divinylbenzene as major components.

(16) The process of producing high purity terephthalic acid as forth above in any of (1) to (15), which is characterized by feeding water or the secondarily separated mother liquor into the adsorption tower in which the desorption step (III) has been finished to desorb the adsorbed desorbing agent.

(17) The process of producing high purity terephthalic acid as set forth above in any one of (1) to (16), which is characterized in that with the adsorption tower in the adsorption step (III) is composed of plural adsorption towers disposed in series; that the requirement of the stopping step (II) is applied to a first tower of the adsorption towers disposed in series; that after stopping the into the first tower, the secondary mother liquor is fed into a second tower of the adsorption towers disposed in series; and the second tower is used as the first tower.

### ADVANTAGES OF THE INVENTION

According to the invention, since p-toluic acid in wastewater which has hitherto been discarded is selectively recovered and used as a raw material of terephthalic acid, it is possible to increase a yield of terephthalic acid. Also, as a result of recovering p-toluic acid from wastewater, since a concentration of organic substances in the wastewater can be lowered, it is possible to reduce a cost required for the wastewater treatment.

### BRIEF DESCRIPTION OF THE DRAWINGS

[Fig. 1] Fig. 1 is a flow diagram to show an embodiment of a process of producing high purity terephthalic acid according to the invention.
[Fig. 2] Fig. 2 is a graph to show concentrations of p-toluic acid and benzoic acid in a feed liquid into an adsorption tower and an effluent in Example 1.
[Fig. 3] Fig. 3 is a graph to show concentrations of p-toluic acid and benzoic acid in a desorbing agent flown out from an adsorption tower in Example 1.
[Fig. 4] Fig. 4 is a graph to show a concentration of acetic acid in an effluent in regenerating an adsorption tower in Example 1.
[Fig. 5] Fig. 5 is a graph to show concentrations of p-toluic acid and benzoic acid in a feed liquid into an adsorption tower and an effluent in Example 2.
[Fig. 6] Fig. 6 is a graph to show concentrations of p-toluic acid and benzoic acid in a desorbing agent flown out from an adsorption tower in Example 2.

### DESCRIPTION OF REFERENCE NUMERALS AND SIGNS

1: Oxidation reaction step
2: Crystallization step
3: Crude terephthalic acid crystal obtaining step
4: Drying step in crude terephthalic acid crystal obtaining step
5: Acetic acid dehydration step
6: Step of forming a slurry of crude terephthalic acid crystal by using water and then dissolving it
7: Hydrogenation step
8: High purity terephthalic acid crystallization step
9: High purity terephthalic acid crystal obtaining step
10: Washing of high purity terephthalic acid crystal
11: Drying of high purity terephthalic acid crystal
12: Crystallization and separation of primary mother liquor
13: Adsorption tower
14: Condensate
15: High purity terephthalic acid crystal
16: Primary mother liquor
17: Secondary crystal
18: Secondary mother liquor
19: Effluent from adsorption tower in feeding a liquid to be treated in adsorption tower
20: Desorbing agent flown out from adsorption tower
21: Regenerated water flown out from adsorption tower
22: Recycle of effluent into dissolving water
23: Recycle of effluent into washing water
24: Recycle of washing wastewater into dissolved solution

### BEST MODES FOR CARRYING OUT THE INVENTION

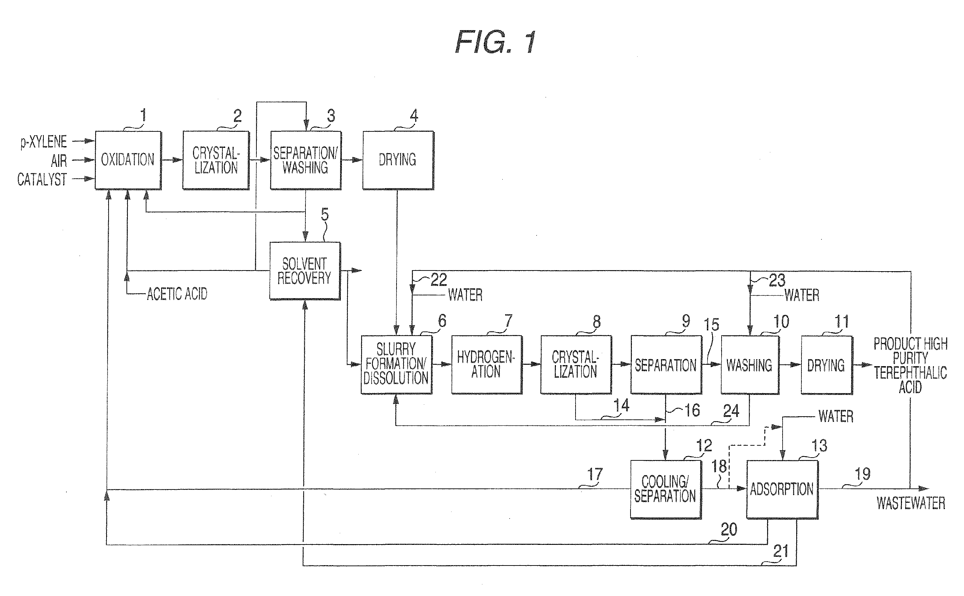

The invention is hereunder described with reference to Fig. 1.

### (a) Oxidation reaction step:

p-Xylene is liquid phase oxidized with molecular oxygen in an acetic acid solvent in the presence of a catalyst, thereby forming terephthalic acid (1). This step is well-known; and catalysts which have hitherto been known to be able to be used in this reaction are used as the catalyst, and specific examples thereof include heavy metal compounds such as cobalt compounds, manganese compounds, iron compounds, and chromium compounds and bromine compounds. These are present in a dissolved state in a reaction system. Of these, combinations of a cobalt compound or a manganese compound and a bromine compound are preferable. In this case, these compounds usually used such that a cobalt atom is from 10 to 5,000 ppm, a manganese atom is from 10 to 5,000 ppm, and a bromine atom is from 10 to 10,000 ppm with respect to the solvent.

In general, a mixed gas of an inert gas and oxygen is used as the molecular oxygen, and for example, air or oxygen-rich air is useful. A molar ratio of the molecular oxygen to p-xylene to be fed into a reactor is usually from 3 to 20 times by mole, and preferably from 2 to 4 times by mole.

A ratio of p-xylene to acetic acid to be fed into a reactor is usually from 1 to 50% by weight. A water concentration in the reaction system is usually from 5 to 20% by weight, and preferably from 5 to 15% by weight.
A temperature of the oxidation reaction is usually from 160 to 260°C, and preferably 170 to 210°C; a pressure is sufficiently a pressure at which the reaction system is able to keep a liquid phase at the reaction temperature or more and is usually from 0.5 to 5 MPa, and preferably from 1 to 2 MPa; and a residence time is usually from 10 to 200 minutes. Though a single reactor is usually used as the reactor, two or more reactors can be connected in series and used, too.

### (b) Crude terephthalic acid crystal obtaining step:

Since terephthalic acid is sparingly soluble in acetic acid as a solvent, terephthalic acid formed in the oxidation reaction step is usually deposited as a crystal to form a slurry. However, there is a possibility that terephthalic acid is dissolved depending upon an amount of the solvent, a reaction temperature and a pressure. In this case, terephthalic acid is deposited by providing a crystallization step (2) of cooling a reaction solution of 160°C to 260°C and from 0.5 to 5 to 90 to 160°C and 0 to 0.2 MPa or the like, thereby forming a slurry. This slurry is subjected to solid-liquid separation (3), thereby obtaining a crude terephthalic acid (hereinafter sometimes abbreviated as "CTA") crystal. Though the terephthalic acid slurry obtained in the oxidation reaction step is in a pressurized state, it may be subjected to solid-liquid separation as it stands or may be subjected to solid-liquid separation after pressure release for cooling or the like. As a method of the solid-liquid separation, any method capable of separating the crystal and the mother liquor from each other is employable, and examples thereof include filtration and centrifugation.

The pressure release for cooling as referred to herein means that by introducing a terephthalic acid slurry into a crystallization tank kept under a pressure condition lower than a pressure of the terephthalic acid slurry obtained in the oxidation reaction step and then releasing the pressure, the slurry temperature is decreased due to expansion and evaporation of the solvent component.

If desired, the thus obtained crude terephthalic acid crystal is washed and dried at from 100 to 200°C (4).
On the other hand, the mother liquor from which the crude terephthalic acid crystal has been separated is usually separated into acetic acid and water by distillation, especially azeotropic distillation in a distillation tower having a reflux ratio of from 0.2 to 10 and a theoretical plate number of from 25 to 125 (acetic acid dehydration step 5). Though recovered acetic acid is circulated into the oxidation reaction step, it may also be used as a desorbing agent in the p-toluic acid recovery step. Since this mother liquor contains methyl acetate formed by a heterogeneous reaction of acetic acid, it is preferable that this is separated and recovered in the distillation. Recovered methyl acetate can be used as the desorbing agent in the p-toluic acid recovery step.

### (c) Hydrogenation step:

In the crude terephthalic acid crystal obtained in the crude terephthalic acid crystal obtaining step, 4-carboxybenzaldehyde (herein sometimes abbreviated as "4CBA") as an oxidation intermediate is contained as an impurity. Since 4C becomes a cause for coloration of terephthalic acid, it must be removed. In this step, 4CBA is converted to p-toluic acid through hydrogenation. Since p-toluic acid is more soluble in water than terephthalic acid, terephthalic acid and p-toluic acid can be easily separated by sequential solid-liquid separation.

In the hydrogenation step, the crude terephthalic acid crystal is heated together with water, thereby completely dissolving crude terephthalic acid in water (6); and 4CBA is then reduced to p-toluic acid through a reaction with hydrogen in the presence of a hydrogenation catalyst (7).

This hydrogenation is also well-known; and catalysts of a metal of the groups 8 to 10, for example, ruthenium, rhodium, palladium, platinum, and osmium useful as the hydrogenation catalyst and usually as a fixed upon being supported on a carrier such as active carbon. Of these, palladium supported on active carbon is preferable.

The crude terephthalic acid crystal is dissolved in a proportion of usually from 1 to 80 parts by weight, and preferably from 15 to 65 parts by weight based on 100 parts by weight of water. A temperature of the hydrogenation is usually from 260 to 320°C, and preferably from 270 to 300°C; and a partial pressure of hydrogen is usually from 0.5 to 20 kg/cm²G.

### (d) High purity terephthalic acid crystallization step:

Since 4CBA in the aqueous solution is reduced to water-soluble p-toluic acid by the hydrogenation step, by crystallizing a terephthalic acid crystal from this, a high purity terephthalic acid crystal free from p-toluic acid is obtained.

As a crystallization method (8), a pressure release for cooling system in which a pressure is dropped to evaporate the solvent is generally employed. Usually, from 2 to 6 crystallization cans are connected in series, and the pressure is dropped stepwise, thereby achieving the crystallization. Usually, a pressure of 6.0 to 12 MPa is dropped to a pressure of 0.1 to 1.0 MPa through from 2 to 6 stages. A final temperature of the crystallization is usually 100°C or more, and preferably from 150 to 160°C. A water vapor generated from the crystallization can is usually cooled and recovered as a condensate (14). Entrained p-toluic acid is frequently contained in this condensate. Thus, it is preferable that this condensate is as a liquid to be treated of an adsorption step (I) as described later, thereby recovering p-toluic acid.

### (e) High purity terephthalic acid crystal obtaining step:

The high purity terephthalic acid slurry obtained in the crystallization step is subjected to solid-liquid separation (9), thereby obtaining a high purity terephthalic acid crystal (hereinafter sometimes abbreviated as "PTA") (15) and a primary mother liquor (16). As a solid-liquid separation method, any method capable of separating a crystal and a mother liquor from each other is employable, and examples thereof include filtration and centrifugation.

Since p-toluic acid is soluble in water, it retains in the primary mother liquor. For that reason, a content of p-toluic acid of the recovered high purity terephthalic acid crystal is extremely low. When the high purity terephthalic acid crystal separated from the primary mother liquor is washed with water (10) and then dried at from 100 to 200°C (11), a high purity terephthalic acid product is obtained. Washing wastewater (24) can be used as water for dissolving the crude terephthalic acid crystal in the hydrogenation step.

On the other hand, water-soluble reaction by-products such as p-toluic acid and benzoic acid and organic substances such as terephthalic acid which has not deposited contained in the primary mother liquor.
In reusing the effluent from the adsorption tower as dissolving water or washing water, it is preferable that the amount for reuse is adjusted, thereby controlling the concentration of benzoic acid in the primary mother liquor at usually not more than 3,000 ppm, and preferably not more than 1,500 ppm. When the concentration of benzoic acid in the primary mother liquor is not excessively high, it is possible to keep the concentration of benzoic acid in a high purity terephthalic acid product obtained by usual washing and drying at not more than 50 ppm, and especially not more than 25 ppm even by recycling the effluent containing benzoic acid. High purity terephthalic acid having a concentration of benzoic acid falling within the foregoing range is able to keep physical properties of high purity terephthalic acid such that a of polymerization of a polyester produced by using this high purity terephthalic acid reaches a prescribed value and that a less colored polyester can be obtained. When the concentration of benzoic acid in the primary mother liquor is too high, in order to obtain high purity terephthalic acid having a content of benzoic acid falling within the foregoing range, washing must be strengthened, the amount of water to be used for washing increases, and a cost required for producing high purity terephthalic acid increases.

### (f) p-Toluic acid recovery step:

In the invention, p-toluic acid in the primary mother liquor is separated from benzoic acid and recovered by using an adsorbing agent (13). The primary mother liquor contains usually from about 500 to 5,000 ppm of p-toluic acid, usually from about 50 to 500 ppm of benzoic acid and usually from about 1,000 to 5,000 ppm of terephthalic acid, the matter of which, however, varies with the crystallization temperature of high purity terephthalic acid.

Though the primary mother liquor can be provided for an adsorption treatment as it stands, it is preferable that this is cooled to deposit a part of dissolved p-toluic acid, terephthalic acid and other organic substances, followed by solid-liquid separation through filtration or other method, thereby separating a secondary crystal (17) and a secondary mother liquor (18) from each other; and that the recovered secondary mother liquor is provided for an adsorption treatment. A cooling temperature of the primary mother liquor is usually from 20 to 100°C, an preferably from 40 to 80°C, and the secondary mother liquor is usually subjected to an adsorption treatment at a temperature falling within this range. In this way, even an adsorbing agent which when brought into contact with the primary mother liquor of a high temperature, is possibly deteriorated can be used in safety. In the secondary mother liquor, since the concentration of p-toluic acid is lowered, a larger amount of the mother liquor can be into the adsorption tower until the adsorption operation is finished. In the case where the primary mother liquor is cooled to room temperature, the secondary mother liquor contains usually from about 100 to 1,000 ppm of p-toluic acid, usually from about 100 to 1,000 ppm of benzoic acid and usually from about 1 to 500 ppm of terephthalic acid. Since a deposit recovered by the solid-liquid separation is composed mainly of p-toluic acid and terephthalic acid, it can be fed into the oxidation reaction step (17).

As described previously, since the condensate (14) obtained by condensing a water vapor generated from the crystallization can in the high purity terephthalic acid crystallization step frequently contains entrained p-toluic acid, this condensate may also be fed into the adsorption tower together with the primary mother liquor or the secondary mother liquor. On that occasion, since the condensate has a high temperature, it is preferred to mix it with the primary mother liquor. The condensate contains usually from about 100 to 1,000 ppm of p-toluic acid, usually from about 10 to 500 ppm of benzoic acid and usually from about 500 to 3,000 ppm of terephthalic acid.

### (I) Adsorption step and (II) feed stopping step:

In the invention, a mixture of the primary mother liquor or the secondary mother liquor or the both and the p-toluic acid-containing condensate (this mixture will be hereinafter referred to as "liquid to be treated") is fed into an adsorption tower (13) filled with an adsorbing agent in which the breakthrough time of p-toluic acid is longer than the breakthrough time of benzoic acid and made to flow down within the tower. In circulating a liquid containing a substance to be adsorbed into the adsorption tower filled with an adsorbing agent, a point of time at which the substance to be adsorbed is detected in the effluent from the adsorption tower is referred to as a breakthrough point of the substance to be adsorbed; and a time from start of the circulation of a liquid containing a substance to be adsorbed into the adsorption tower to arrival at the breakthrough point of the substance to be adsorbed is referred to as a breakthrough time of the substance to be adsorbed.

Since benzoic acid and p-toluic acid in the liquid to be treated are adsorbed to the adsorbing agent, a liquid which does not contain these flows out from an end of the adsorption tower (19). However, when the feed of the liquid to be treated is continued, it reaches the breakthrough point of benzoic acid, whereby benzoic acid becomes contained in the liquid flown out from the end of the adsorption tower. Here, when the solution containing p-toluic acid and benzoic acid is treated by the adsorption tower, a point at which benzoic acid is detected in the effluent is referred to as a breakthrough point of benzoic acid, and a time required from start of the liquid circulation to arrival at the breakthrough point is referred to as a breakthrough time. When the feed of the liquid to be treated into the adsorption tower is continued exceeding the breakthrough time of benzoic acid, a concentration of benzoic acid in the liquid flown out from the end increases gradually and after a while, becomes higher than a concentration of benzoic acid in the liquid to be treated. This reveals that p-toluic acid in the liquid to be treated displaces benzoic acid adsorbed to the adsorbing agent and is adsorbed.

When the of the liquid to be treated is further continued, it reaches the breakthrough point of p-toluic acid, and p-toluic acid becomes also contained in the liquid flown out from the end. Here, when the solution containing p-toluic acid and benzoic acid is treated by the adsorption tower, a point at which p-toluic acid is detected in the effluent is referred to as a breakthrough point of p-toluic acid, and a time required from start of the liquid circulation to arrival at the breakthrough point is referred to as a breakthrough time. When the feed of the liquid to be treated into the adsorption tower is continued exceeding the breakthrough time of p-toluic acid, a concentration of p-toluic acid in the liquid increases gradually and becomes finally equal to the concentration of the liquid to be treated. Until arrival at the breakthrough point of benzoic acid (breakthrough time of benzoic acid), a ratio of p-toluic acid to benzoic acid within the adsorption tower is equal to a ratio of p-toluic acid to benzoic acid in the liquid to be treated; however, when it exceeds the breakthrough point, this ratio increases gradually, and at a point of time at which concentrations of benzoic acid and p-toluic acid in the effluent correspond with those in the liquid to be treated, namely at a saturation point, this ratio becomes maximum. Accordingly, when the feed of the liquid to be treated into the adsorption tower is stopped at an arbitrary point of time after passing through the breakthrough point of benzoic acid (breakthrough time of benzoic acid) until arrival at the saturation point, and instead, a desorbing agent is fed to adsorbed benzoic acid and p-toluic acid, it is possible to recover p-toluic acid having a ratio of p-toluic acid to benzoic acid higher than that in the liquid to be treated.

Usually, the feed of the liquid to be treated is stopped after the concentration of benzoic acid in the effluent has reached at least 10%, and especially at least 20% of the concentration in the liquid to be treated. In general, it is preferable that the feed is stopped after the concentration of benzoic acid in the effluent has reached 50% or more, and especially 100% or more of the concentration in the liquid to be treated. When the feed of the liquid to be treated is stopped after p-toluic acid has become contained in the effluent, it is possible to recover p-toluic acid having a smaller content of benzoic acid. When the concentration of p-toluic acid in the effluent at a point of time of stopping the feed is high, though the concentration of benzoic acid in recovered p-toluic acid is lowered, the recovery amount of p-toluic acid is inversely reduced. Though a point of time at which the feed of the liquid to be treated should be stopped is determined while taking into consideration a recovery of p-toluic acid and a tolerable amount of benzoic acid incorporated into p-toluic acid, the feed is usually stopped before the concentration of p-toluic acid in the effluent reaches 90%, and preferably 50% of p-toluic acid in the liquid to be treated. In general, it is preferable that the feed is stopped at a point of time at which it has reached at least 80%, and especially at least 90% of the breakthrough time of p-toluic acid and during a time from the breakthrough point of p-toluic acid to arrival at a concentration of p-toluic acid in the effluent of 25%, preferably 20%, and especially 10% in the liquid to be treated.

Though an arbitrary adsorbing agent can be used so far as it is a porous adsorbing agent in which the breakthrough time of benzoic acid is longer than the breakthrough time of p-toluic acid, an organic synthetic adsorbing agent is usually used. For example, styrene-divinylbenzene based synthetic adsorbing agents such as SEPABEADS SP825, SP850 and SP207 (SEPABEADS is a registered trademark of Mitsubishi Chemical Corporation) and AMBERLITE XAD-4 and XAD-16 (AMBERLITE is a registered trademark of Rohm and Haas Company) and acrylic synthetic adsorbing agents such as DIAION HP2MG (DIAION is a registered trademark of Mitsubishi Chemical Corporation) and AMBERLITE XAD-7 and XAD-8 can be used. Preferably, non-polar organic synthetic adsorbing agents, especially synthetic adsorbing agents composed of a porous copolymer of a monovinyl compound and a polyvinyl compound, and above all, styrene-divinylbenzene based synthetic adsorbing agents are used. Since both benzoic acid and p-toluic acid contain a benzene ring, they are easily adsorbed to a styrene-divinylbenzene based synthetic agent, and an adsorptivity of p-toluic acid is generally larger than that of benzoic acid.

A specific surface area of the adsorbing agent is usually from 400 to 1,500 m²/g, and preferably from 600 to 1,000 m²/g; its pore volume is usually from 0.5 to 3 mL/g, and preferably from 1.0 to 2.0 mL/g; and its pore size is usually from 10 to 1,000 angstroms, and preferably from 50 to 500 angstroms. The adsorbing agent is filled such that a height of the filling layer is usually from about 1.5 to 4.0 m. In general, when the height of the filling layer is too low, a use efficiency of the adsorbing agent is lowered, the matter of which, however, varies with a point of time at which the feed of the liquid to be treated should be stopped. A feed rate of the liquid to be treated into the adsorption tower is usually from 0.5 to 30 m/hr for LV and usually from 0.5 to 20 hr⁻¹ for SV, respectively.

### (III) Desorption step:

After stopping the feed of the liquid to be treated into the adsorption tower, a desorbing agent is subsequently fed into the adsorption tower (not illustrated), thereby desorbing adsorbed benzoic acid and p-toluic acid. After stopping the feed of the liquid to be treated, the desorbing agent may be fed after feeding water and discharging the liquid to be within the adsorption tower.

Acetic acid, methyl acetate or a mixture thereof may be used as the desorbing agent. Since acetic acid or methyl acetate is recovered from a vapor generated within the system or a mother liquor of the crude terephthalic acid crystal during a time from the oxidation reaction step to the crude terephthalic acid crystal obtaining step, this can be used.

A feed rate of the desorbing agent is usually from 0.5 to 30 m/hr for LV and usually from 0.5 to 20 hr⁻¹ for SV, respectively. A temperature of the desorbing agent may be arbitrarily chosen within the range of a freezing point or higher and not higher than a boiling point of the desorbing agent; and for example, in the case where the desorbing agent is acetic acid, a temperature of from room temperature to 80°C is preferable. Though a ratio of p-toluic acid and benzoic acid in the desorbing agent solution containing p-toluic acid and benzoic acid flown out from the adsorption tower varies with a proportion of p-toluic acid and benzoic acid in the liquid to be treated and point of time at which the feed of the liquid to be treated is stopped, it is preferable that a ratio of benzoic acid to p-toluic acid is not more than 35% by weight.

### (IV) Circulation step:

p-Toluic acid in the desorbing agent (20) flown out from the adsorption tower, usually p-toluic acid and benzoic acid are fed into the oxidation reaction step. Simply, the flown out desorbing agent can be fed into the oxidation reaction step as it stands. In the case where acetic acid is used as the desorbing agent, it is preferable that the flown out desorbing agent is brought into contact with a reaction waste gas generated in the oxidation reaction step and then fed into the oxidation reaction step. Though methyl acetate generated from acetic acid as a reaction solvent is contained in the reaction waste gas, since this is absorbed on acetic acid and returned to the oxidation reaction system, it is possible to suppress a reaction from acetic acid to methyl acetate.

Next, water is fed into the adsorption tower after passing through the desorption step to discharge the desorbing agent within the tower, thereby regenerating the adsorption tower. A feed rate of water is usually from 0.5 to 30 m/hr for LV and usually from 0.5 to 20 hr⁻¹ for SV, respectively. Though a temperature of water to be fed is arbitrary, it is usually from room temperature to 80°C. Since the desorbing agent is contained in regenerated water (21) flown out from the adsorption tower, for example, a liquid containing 50% or more of acetic acid at the beginning of regeneration is fed into the oxidation reaction system; and regenerated water in the sequential steps is fed as a reflux liquid of distillation, etc. for distillation in an acetic acid dehydration step and distilled, thereby recovering the desorbing agent and reusing it. The distillation can be achieved together with an acetic acid aqueous solution generated within the system, in addition to single distillation. Here, the secondary mother liquor can also be used as water to be fed into the adsorption tower.

Though the invention can be carried out by using a single adsorption tower, in the adsorption step (I), it is possible to enable one to achieve the adsorption treatment continuously as a whole by providing plural adsorption towers filled with an adsorbing agent in which a breakthrough time of p-toluic acid is longer than a breakthrough time of benzoic acid and successively switching the adsorption tower to which the liquid to be treated is fed. Each of the adsorption towers is configured so as to repeat adsorption - desorption - regeneration. A "primary mother liquor or secondary mother liquor obtained by subjecting the primary mother liquor as cooled to solid-liquid separation" is fed as the liquid to be treated into a first adsorption tower, and p-toluic acid and benzoic acid in the liquid to be treated are adsorbed to the adsorbing agent. At this time, though the effluent of the first adsorption tower is discharged out an adsorption process system during a time when p-toluic acid does not flow out in the effluent, this effluent can be reused as water for washing high purity terephthalic acid in a high purity terephthalic acid crystal washing step (g) or dissolving the crude terephthalic acid crystal in the hydrogenation step (c). Then, in the stropping step (II), when p-toluic acid in the effluent from the first adsorption tower has reached a prescribed concentration, by stopping the feed of the liquid to be treated into the first adsorption tower and switching a destination of the liquid to be treated from the first adsorption tower to a second adsorption tower, a recovery of p-toluic acid in the liquid to be treated is not lowered. Accordingly, in this case, at a point of time at which a ratio of p-toluic acid to benzoic acid in the first adsorption tower becomes the highest, the feed of the liquid to be treated into the first adsorption tower can be stopped. In the desorption step (III), a desorbing agent is fed into the first adsorption tower to desorb adsorbed p-toluic acid; and in the circulation step (IV), p-toluic acid contained in the desorbing agent flown out from the first adsorption tower is fed into the oxidation reaction step.

In the invention, a plural number (usually two) of adsorption towers may be disposed in series, and on that occasion, the requirements (I) to (IV) in the invention of this application may be applied and operated in a first tower of the adsorption towers disposed in series. After stopping the feed of the liquid to be treated into the first tower, by feeding this liquid to be treated into a second tower of the adsorption towers disposed in series, this second tower may be used as the first tower. In using the second tower as the first tower, a new final tower in which the regeneration has been finished (for example, in the of disposing two towers in series, a new second tower) may be provided.

### (g) High purity terephthalic acid crystal washing and drying step:

By washing the high purity terephthalic acid crystal separated from the primary mother liquor with water (10) and then drying it (11), a high purity terephthalic acid product is obtained. The washing of the high purity terephthalic acid crystal may be carried out by a method of feeding water into a solid-liquid separation apparatus or by a method of suspension washing the high purity terephthalic acid crystal with water, followed by achieving solid-liquid separation. Here, as the solid-liquid separation apparatus, the apparatus as used in the foregoing solid-separation operation can be used. As one preferred example of the solid-liquid separation apparatus, a screen bowl type centrifuge and the like are enumerated.

In the case where the washing is carried out by feeding water into the solid-liquid separation apparatus, a temperature of water which is used for washing is usually a temperature of the high purity terephthalic acid slurry obtained in the crystallization step or higher and not higher than a boiling point under an operation pressure, preferably from 100 to 180°C, and more preferably from 140 to 170°C. In the case of carrying out the suspension washing, a temperature of water which is used for washing is usually from 30 to 180°C. Though an amount of water to be used for such washing varies with an amount of impurities contained in high purity terephthalic acid, a tolerable amount of impurities for product high purity terephthalic acid, a washing method and the like, water is usually used in an amount of from about 0.1 to 2 tons per ton of the high purity terephthalic acid crystal. A dryer such as a steam tube type dryer and a fluidized layer dryer is used for drying, and a drying temperature is usually from 80 to 180°C.

### (h) Washing wastewater circulation step:

At least a part of washing wastewater in the high purity terephthalic acid crystal washing and drying step is reused as water for dissolving the crude terephthalic acid crystal in the hydrogenation step (c) (24). Preferably, the whole amount of washing wastewater is used as dissolving water.

In feeding the liquid to be treated into the adsorption tower, since a liquid flown out from the adsorption tower (19) does not substantially contain organic substances until arrival at the breakthrough point of benzoic acid (breakthrough time of benzoic acid), among the effluents after the breakthrough point of benzoic acid, a part or the whole, and preferably a part of one having a low benzoic acid concentration can be used as dissolving water (22) of the crude terephthalic acid crystal in the hydrogenation step or washing water (23) of the high-purity terephthalic acid crystal after the solid-liquid separation in the high purity terephthalic acid crystal obtaining step (e). In the case of use as washing water in the recovery of the high purity terephthalic acid crystal, washing wastewater is usually used as dissolving water of crude terephthalic acid in the hydrogenation step (24).

Thus, in the case of reusing the effluent from the adsorption tower as dissolving water or washing water, by adjusting the amount for reuse so as to have a benzoic acid concentration in the primary mother liquor of usually not more than 3,000 ppm, and preferably not more than 1,500 ppm, it is possible to keep a benzoic acid concentration in the resulting high purity terephthalic acid product at not more than 60 ppm, and especially not more than 30 ppm; a degree of polymerization of a polyester produced by using this reaches a prescribed value; and a less colored polyester can be obtained.

Among the adsorption effluents, one which is not used within the system is discarded. However, heavy metals derived from the catalyst in the oxidation reaction step are contained in the liquid, and when this is discharged into the environment, there is a. possibility that it adversely affects the environment. Accordingly, it is preferred to discharge the liquid after removing the heavy metals through a treatment with an ion exchange resin. A usual strongly acidic ion exchange resin may be used as the ion exchange resin. The heave metals adsorbed to the ion exchange resin can be reused as a catalyst in the oxidation reaction step through desorption with an acid.

The invention is hereunder described specifically with reference to the Examples, but it should be construed that the invention is never limited to Examples.

### EXAMPLE 1

18.8 g (33 mL) of a synthetic adsorbing agent SEPABEADS SP825L, manufactured by Mitsubishi Chemical Corporation was weighed in a beaker and formed into a slurry by using methanol; and after thoroughly degassing, the slurry was poured into a glass column having an inside diameter of 17.8 mm. The slurry was washed with pure water, and it was confirmed by gas chromatography that methanol had not be detected.

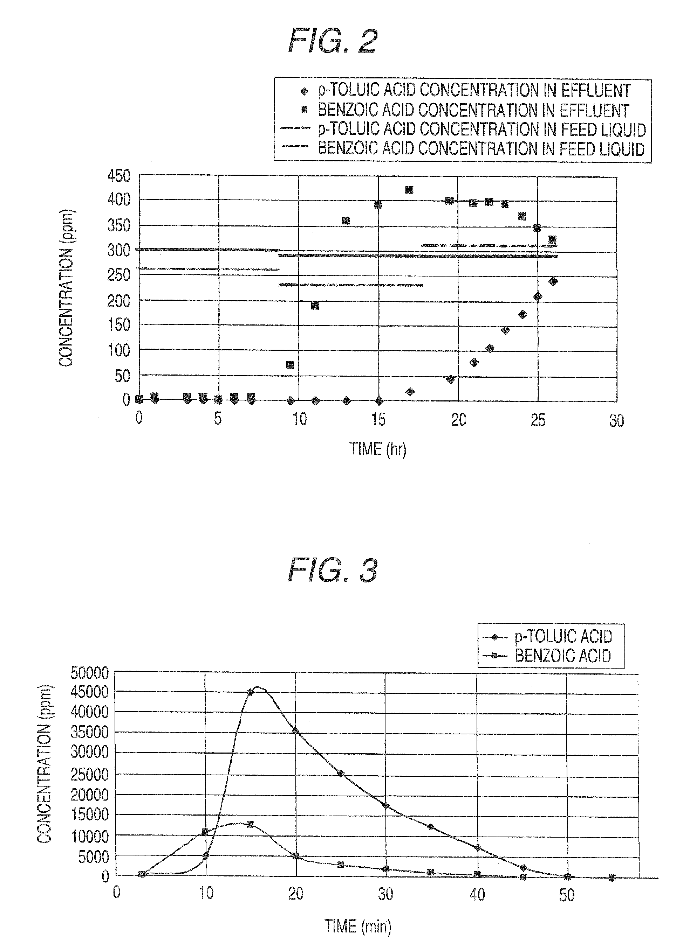

An aqueous solution containing p-toluic acid and benzoic acid was continuously fed into this column at 338 mL/hr, thereby achieving an adsorption treatment. The feed was carried out at SV = 10 hr⁻¹ and LV = 1.4 m/hr. Concentrations of p-toluic acid and benzoic acid in a feed liquid and an effluent were periodically measured. The results shown in Fig. 2.

Next, acetic acid containing 300 ppm of p-toluic acid and 7% of water was fed as a desorbing agent at SV = 10 hr⁻¹ into the foregoing column. Concentrations of p-toluic acid and benzoic acid in the desorbing agent flown out from the column were periodically measured. The results are shown in Fig. 3.

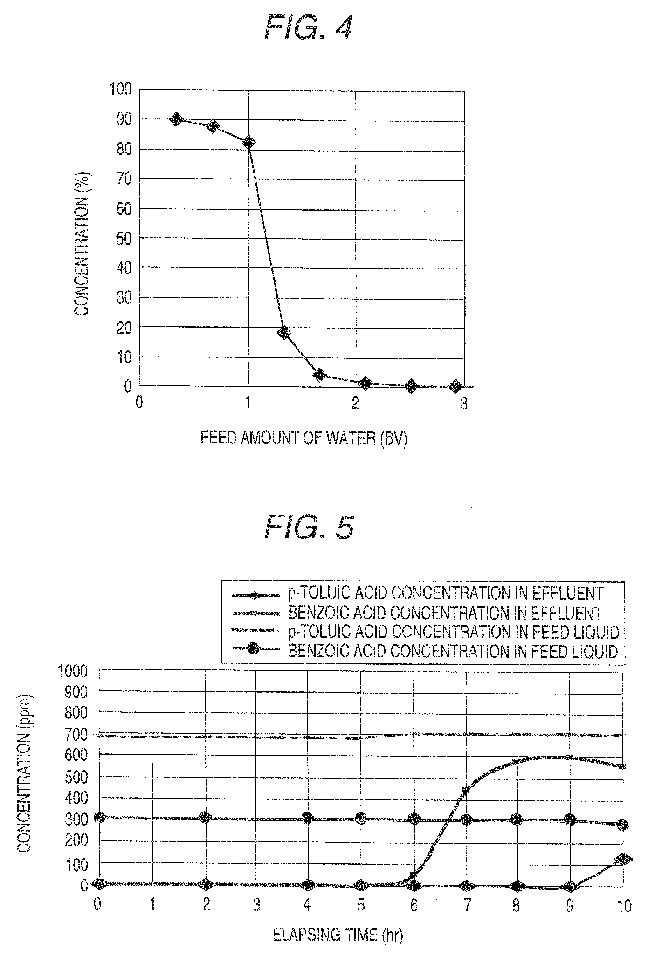

Also, water having a room temperature was provided at SV = 5 hr⁻¹ to the column desorbed with acetic acid to regenerate the column while concentration of acetic acid in water flown out was periodically measured. The results shown in Fig. 4.

### EXAMPLE 2

A crude terephthalic acid slurry obtained by oxidizing p-xylene with air in an acetic acid solvent in the presence of a catalyst composed of a cobalt compound, a manganese compound and hydrogen bromide was subjected to solid-liquid separation to obtain a crude terephthalic acid crystal. An aqueous slurry containing this crude terephthalic acid crystal in a proportion of 30% by weight was heated to prepare an aqueous solution, which was then hydrogenated at 290°C and 8.7 in the composed of a catalyst of palladium supported on active carbon.

The hydrogenated aqueous solution was subjected to pressure release for cooling stepwise by a crystallization tank having five crystallization cans connected to each other in series and finally cooled to 155°C, thereby obtaining a high purity terephthalic acid slurry. This slurry was subjected to solid-liquid separation to obtain a high purity terephthalic acid crystal and a primary mother liquor. The high purity terephthalic acid crystal was washed with water in an amount of 1.5 tons per ton of high purity terephthalic acid and then dried at 140°C, thereby obtaining a high purity terephthalic acid product having a benzoic acid concentration of 5 ppm. The primary mother liquor was subjected to pressure release for cooling to 80°C in a tank of pressure release for cooling, thereby depositing a crystal and forming a slurry. This was separated by a filter to obtain a secondary crystal and a secondary mother liquor. The secondary mother liquor had a benzoic acid concentration of 300 ppm and a p-toluic acid concentration of 700 ppm.

13 kg (19 L) of a synthetic adsorbing agent SEPABEADS SP207, manufactured by Mitsubishi Chemical Corporation was charged in a stainless steel-made column having an inside diameter of 260 mm, water was added, and degassing was thoroughly carried out to complete an adsorption tower. The secondary mother liquor was continuously fed into this adsorption tower at SV = 10 hr⁻¹ and LV = 3.6 m/hr. Concentrations of benzoic acid and p-toluic acid in a feed liquid into the adsorption tower and an effluent from the adsorption tower are shown in Fig. 5. The feed liquid contains about 80 ppm of terephthalic acid.

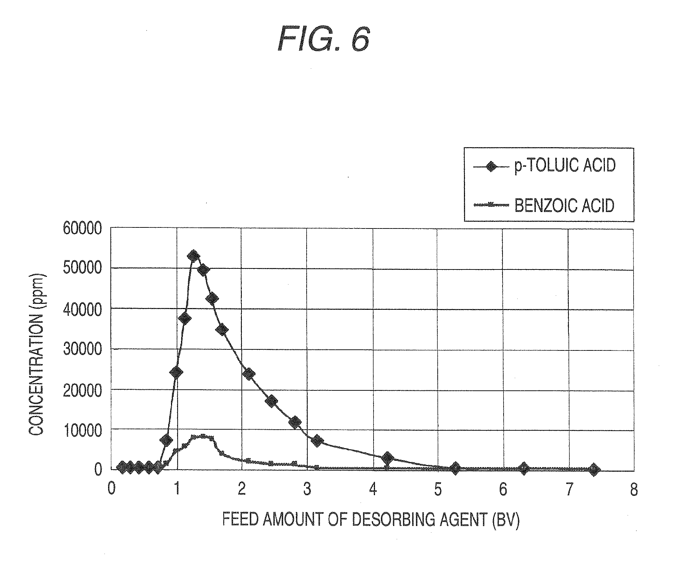

Acetic acid containing 300 ppm of p-toluic acid and 7% of water was fed at SV = 2 hr⁻¹ into the adsorption tower having benzoic acid and p-toluic acid adsorbed therein, thereby desorbing adsorbed benzoic acid and p-toluic acid. The results shown in Fig. 6.

It is noted from this phenomenon that in feeding the aqueous solution containing both benzoic acid and p-toluic acid into the adsorption tower filled with this porous adsorbing agent, judging from the effluence state of these compounds into an effluent flown out from the adsorption tower, at the beginning, all of benzoic acid and p-toluic acid are adsorbed and therefore, a liquid not containing any of the both flows out from the adsorption tower; however, when the feed of the aqueous solution is continued, benzoic acid becomes contained in the effluent and the concentration of benzoic acid in the effluent increases gradually, and when this concentration becomes considerably high, p-toluic acid becomes first contained in effluent and its concentration increases gradually.

This reveals that p-toluic acid is stronger in adsorptivity to the porous adsorbing agent than benzoic acid and that after the adsorption tower has reached substantially saturated adsorption by benzoic acid and p-toluic acid, p-toluic acid displaces adsorbed benzoic acid and is adsorbed. Accordingly, it is noted that by utilizing this phenomenon and stopping the feed of the aqueous solution at a point of time at which adsorbed benzoic acid is thoroughly displaced with p-toluic acid and a ratio of p-toluic acid to benzoic acid within the adsorption tower is thoroughly larger than the ratio in the feed liquid, p-toluic acid can be selectively recovered.

By feeding acetic acid, an oxidation reaction solvent, into this adsorption tower having benzoic acid and p-toluic acid adsorbed therein, it is possible to desorb adsorbed benzoic acid and p-toluic acid, and therefore, terephthalic acid can be advantageously produced by circulating p-toluic acid which is useful as a raw material of terephthalic acid into the oxidation reaction step.

### EXAMPLE 3

0.5 g of a synthetic adsorbing agent SEPABEADS SP825, manufactured by Mitsubishi Chemical Corporation was added in 100 of an aqueous solution containing p-toluic acid in a concentration of 332 ppm, and the mixture was stirred at room temperature for 15 hours, thereby equilibrium adsorbing p-toluic acid. An amount of p-toluic acid adsorbed to the adsorbing agent was 27 mg.

This adsorbing agent was taken out from the aqueous solution, to which was then added 20 mL of methyl acetate, and the mixture was stirred at room temperature for 6 hours to desorb p-toluic acid. As a result, an amount of p-toluic acid in the desorbing agent was 27 mg.
This adsorbing agent was washed with 100 mL of water and then added in 100 mL of aqueous solution containing p-toluic acid in a concentration of 332 ppm, and the mixture was stirred at room temperature for 15 hours to again adsorb p-toluic acid. As a result, an amount of p-toluic acid adsorbed to the adsorbing agent was 23 mg, and it was confirmed that methyl acetate is appropriate as a desorbing agent.

### EXAMPLE 4

13 kg (19 L) of a synthetic adsorbing agent SEPABEADS SP207, manufactured by Mitsubishi Chemical Corporation was charged in a stainless steel-made column having an inside diameter of 260 mm and a volume of 24 L, and this secondary mother liquor was fed at SV = 10 hr⁻¹ and LV = 3.6 m/hr for 10 hours. When the feed was stopped, a concentration of p-toluic acid in the effluent was 127 ppm. All of the effluents from the adsorption tower were recovered in a tank. As a result, a volume of the effluent was 1,900 L, a benzoic acid concentration was 222 ppm, and a p-toluic acid concentration was 12 ppm.

Concentrations of benzoic acid contained in a primary mother liquor and product high purity terephthalic acid at the time of using the thus obtained effluent as a part of washing water of the high purity terephthalic crystal and using washing wastewater for the whole amount of dissolving water of high purity terephthalic acid were computed by simulation. The results are shown in Table 1.

**[Table 1]**

| | Proportion of liquid to be reused as washing liquid in effluent from adsorption tower (% by weight) | Benzoic acid concentration in primary mother liquor (ppm) | acid concentration in product high purity terephthalic acid (ppm) |
|---|---|---|---|
| Example 1 | 20 | 312 | 6 |
| Example 2 | 50 | 497 | 10 |
| Example 3 | 70 | 821 | 16 |
| Comparative Example 1 | 90 | 2353 | 47 |

### EXAMPLE 5

To high purity terephthalic acid (PTA) manufactured by Mitsubishi Chemical Corporation, benzoic acid was added in an amount corresponding to 0 ppm, 50 ppm and 100 ppm, respectively, and 1.004 parts by weight based on part by weight of terephthalic acid of ethylene glycol was charged, and the mixture was esterified at 235°C for 53 minutes and further at 260°C for 45 minutes and then polymerized at 280°C for 100 minutes in polymerization evaluation equipment. A Hunter b value of the resulting polyester was measured by a color difference meter, manufactured by Suga Test Instruments Co., Ltd. As a result, the Hunter b value was 1.38, 1.68 and 1.94, respectively.

### Consideration:

Oxidation of p-xylene produces p-toluic acid. Oxidation of p-toluic acid produces 4-carboxybenzoic acid, and oxidation of 4-carboxybenzoic acid produces terephthalic acid. For that reason, by feeding p-toluic acid recovered in Examples 1 to 3 into the oxidation step, terephthalic acid is produced, and the yield of terephthalic acid can be enhanced.

While the invention has described in detail and with reference to specific embodiments thereof, it will be apparent to one skilled in the art that various changes and modifications can be made therein without departing from the spirit and thereof.

This application is based on a Japanese patent application filed March 22, 2005 (Japanese Patent Application No. 2005-082038) and a Japanese patent application filed March 23, 2005 (Japanese Patent Application No. 2005-084186), the entire contents thereof being hereby incorporated by reference.

### INDUSTRIAL APPLICABILITY

According to the invention, since p-toluic acid in wastewater which has hitherto been discarded is selectively recovered and used as a raw material of terephthalic acid, it is possible to increase a yield of terephthalic acid. Also, as a result of recovering p-toluic acid from wastewater, since a concentration of organic substances in the wastewater can be lowered, it is possible to reduce a cost required for the wastewater treatment. Thus, an industrial value of the invention is remarkable.

## Claims

1. A process of producing high purity terephthalic acid including respective steps of (a) an oxidation reaction step of oxidizing p-xylene in an acetic acid solution where a catalyst is present to form terephthalic acid, (b) a crude terephthalic acid crystal obtaining step of subjecting a slurry in which formed terephthalic acid is deposited to solid-liquid separation to obtain a crude terephthalic acid crystal, (c) a hydrogenation step of dissolving the crude terephthalic acid crystal in water to form an aqueous solution and hydrogenating this, (d) a high purity terephthalic acid crystallization step of crystallizing terephthalic acid from the hydrogenated aqueous solution to form a high purity terephthalic acid slurry, (e) a high purity terephthalic acid crystal obtaining step of subjecting the high purity terephthalic acid slurry to solid-liquid separation to obtain a high purity terephthalic acid crystal and a primary mother liquor, and (f) a p-toluic acid recovery step of recovering p-toluic acid from the primary mother liquor and feeding it into oxidation reaction step, which is **characterized in that** the p-toluic acid recovery step includes the following respective steps:
(I) an adsorption of feeding, as a liquid to be treated, the primary mother liquor or a secondary mother liquor obtained by subjecting primary mother liquor as cooled to solid-liquid separation into an adsorption tower filled with an adsorbing agent in which the breakthrough time of p-toluic acid is longer than the breakthrough time of benzoic acid to adsorb p-toluic acid and benzoic acid in the liquid to be treated to the adsorbing agent,
(II) a feed stopping step of stopping the feed of the liquid to be treated into the adsorption tower at an arbitrary point of time of exceeding the breakthrough time of benzoic acid,
(III) a desorption step of feeding a desorbing agent into the adsorption tower to desorb adsorbed p-toluic acid, and
(IV) a circulation step of feeding p-toluic acid contained in the desorbing agent flown out from the adsorption tower into the oxidation reaction step.

2. The process of producing high purity terephthalic acid according to claim 1, which is **characterized by** further including the following respective steps:
(g) a high purity terephthalic acid crystal washing and drying step of washing the high purity terephthalic acid crystal with water and then drying it to obtain a high purity terephthalic acid product, and
(h) a washing wastewater circulation step of reusing washing wastewater in the high purity terephthalic acid crystal washing and drying step as water for dissolving the crude terephthalic acid crystal in the hydrogenation step (c).

3. The process of producing high purity terephthalic acid according to claim 1, which is **characterized by** further including the following step:
(i) an effluent circulation step of reusing at least a part of an effluent from the adsorption tower in the adsorption step (I) as water for dissolving the crude terephthalic acid crystal in the hydrogenation step (c).

4. The process of producing high purity terephthalic acid according to claim 2, which is **characterized by** further including the following step:
(i) an effluent circulation step of reusing at least a part of an effluent from the adsorption tower in the adsorption step (I) as water for dissolving the crude terephthalic acid crystal in the hydrogenation step (c).

5. The process of producing high purity terephthalic acid according to claim 2, which is **characterized by** further including the following step:
(ii) an effluent circulation step of reusing at least a part of an effluent from the adsorption tower in the adsorption step (I) as water for washing the high purity terephthalic acid crystal in the high purity terephthalic acid crystal washing step (g).

6. The process of producing high purity terephthalic acid according to claim 3 or 4, which is **characterized by** adjusting an amount of the effluent to be reused in the effluent circulation step (i) such that the concentration of benzoic acid in the primary mother liquor in the high purity terephthalic acid crystal obtaining step (e) is not more than 3,000 ppm.

7. The process of producing high purity terephthalic acid according to claim 5, which is **characterized by** adjusting an amount of the effluent to be reused in the effluent circulation step (ii) such that the concentration of benzoic acid in the primary mother liquor in the high purity terephthalic acid crystal obtaining step (e) is not more than 3,000 ppm.

8. The process of producing high purity terephthalic acid according to claim 1, which is **characterized by** carrying out the crystallization of terephthalic acid in high purity terephthalic acid crystallization step (d) by evaporating water from the aqueous solution to lower the temperature of the aqueous solution, condensing the generated water vapor and feeding this condensate as the liquid to be treated into the adsorption tower in the adsorption step (I).

9. The process of producing high purity terephthalic acid according to claim 1, which is **characterized by** carrying out the feed stopping of the liquid to be treated into the adsorption tower in the feed stopping step (II) at a point of time at which the concentration of benzoic acid in the effluent from adsorption tower has reached at least 50% of the concentration of benzoic acid in the liquid to be treated.

10. The process of producing high purity terephthalic acid according to claim 1, which is **characterized by** carrying out the stopping of liquid to be treated into the adsorption tower in the feed stopping step (II) at or after the point of time at which the concentration of benzoic acid in the effluent from adsorption tower has become equal to the concentration of benzoic acid in the liquid to be treated.

11. The process of producing high purity terephthalic acid according to claim 1, which is **characterized by** carrying out the feed stopping of the liquid to be treated into the adsorption tower in the feed stopping step (II) before the concentration of p-toluic acid in the effluent from adsorption tower reaches 50% of the concentration of p-toluic acid in the liquid to be treated.

12. The process of producing high purity terephthalic acid according to claim 1, which is **characterized by** carrying out feed stopping of the liquid to be treated into the adsorption tower in the feed stopping step (II) before the concentration of p-toluic acid in the effluent from adsorption tower 20% of the concentration of p-toluic acid in the liquid to be treated.

13. The process of producing high purity terephthalic acid according to claim 1, which is **characterized in that** in the adsorption step (I), plural adsorption towers filled with an adsorbing agent in which the breakthrough time of p-toluic acid is longer than the breakthrough time of benzoic acid are provided, and the respective adsorption towers configured so as to repeat adsorption - desorption - regeneration, the primary mother liquor or the secondary mother liquor obtained by subjecting the primary mother liquor as cooled to solid-liquid separation is fed into a first adsorption tower and p-toluic acid and benzoic acid in the liquid to be treated are adsorbed to the adsorbing agent; that in the stropping step (II), when p-toluic acid in the effluent from the first adsorption tower has reached a prescribed concentration, the feed of the liquid to be treated into the first adsorption tower is stopped, and a feed destination of the liquid to be treated is switched from the first adsorption tower to a second adsorption tower; that in the desorption step (III), the desorbing agent is fed into the first adsorption tower to desorb adsorbed p-toluic acid; and in the circulation step (IV), p-toluic acid contained in the desorbing agent flown out from the first adsorption tower is fed into the oxidation reaction step.

14. The process of producing high purity terephthalic acid according to claim 13, which is **characterized by** further feeding water into the first adsorption tower after passing through the desorption step (III) to elute the adsorbed desorbing agent and using the first adsorption tower as the adsorption tower in the adsorption step (I).

15. The process of producing high purity terephthalic acid according to claim 1, which is **characterized in that** the desorbing agent is acetic acid, methyl acetate or a mixture thereof.

16. The process of producing high purity terephthalic acid according to claim 1, which is **characterized in that** the adsorbing agent is a porous copolymer of a monovinyl compound and a polyvinyl compound.

17. The process of producing high purity terephthalic acid according to claim 1, which is **characterized in that** the adsorbing agent is a porous copolymer containing styrene and divinylbenzene as major components.

18. The process of producing high purity terephthalic acid according to claim 1, which is **characterized by** feeding water or the secondary mother liquor into the adsorption tower in which the desorption step (III) has been finished to desorb the adsorbed desorbing agent.

19. The process of producing high purity terephthalic acid according to claim 1, which is **characterized in that** with the adsorption tower in the adsorption step (III) is composed of plural adsorption towers disposed in series; that the requirement of the feed stopping step (II) is applied to a first tower of the adsorption towers disposed in series; that after stopping the feed into the first tower, the liquor to be treated is fed into a second tower of the adsorption towers disposed in series; the second tower is used as the first tower.

20. The process of producing high purity terephthalic acid according to claim 13, which is **characterized by** discharging the effluent of the first adsorption tower out an adsorption process system during a time when p-toluic acid does not flow out in the effluent.
